# EUROPEAN PATENT APPLICATION

(11) **EP 1 508 572 A1**
(43) Date of publication of application: **23.02.2005**
(21) Application number: 03733179.0
(22) Date of filing: 29.05.2003
(51) Int. Cl.: C07K 14/195, C12N 15/31, C12N 9/10, C12Q 1/68, A61P 1/04

(54) **PROTEIN INDUCING CELL DEATH OF i HELICOBACTER PYLORI /i**

(30) Priority: 30.05.2002 JP 2002156768
(71) Applicant: Japan Science and Technology Agency, Kawaguchi-shi, Saitama 332-0012 (JP); Japan as repreesented by President of National Institute of Infectious Diseases, Shinjuku-ku, Tokyo 169-0052 (JP)
(72) Inventor: Arakawa, Yoshichika, Tachikawa-shi, Tokyo 190-0002 (JP); Shibayama, Keigo, Musashimurayama-shi, Tokyo 208-0011 (JP)
(74) Representative: Wilson Gunn Gee
(86) International application number: PCT/JP2003/006787
(87) International publication number: WO 2003/102025

(57) **Abstract**

The present invention identifies the apoptosis-inducing protein of *Helicobacter pylori,* analyzes the structure and provides the amino acid sequence of the protein and the nucleotide sequence encoding the amino acid sequence. Furthermore, the present invention provides inhibiting agents of an apoptosis-inducing activity of the bacterium for the treatment of disease ascribed to the bacterium and a vaccine to prevent the infection of the bacterium.

The present invention revealed that the apoptosis-inducing protein of *Helicobacter pylori* is a protein complex composed by association of two kinds of proteins, which comprise a part of the amino acid sequence encoded by γ-glutamyl transferase gene (HP1118). The present invention is said protein and a vaccine comprising said protein.

## Description

### Field of the Invention

The present invention relates to an apoptosis-inducing protein from *Helicobacter pylori* and, more specifically, relates to two novel proteins constituting the apoptosis-inducing protein.

### Prior Art

*Helicobacter pylori* infects persistently human stomach and has been implicated in the pathogenesis of gastritis and gastric ulcer. *Helicobacter pylori* infection induces apoptosis in gastric epithelial cells, which plays a significant role in the development of the pathological outcomes. Conventionally, an eradication therapy using antibiotics has been applied to the *Helicobacter pylori* infection, however, antibiotics-resistant strains have been emerging and at times the eradication therapy by antibiotics is unsuccessful. Consequently, a new methodology of treatment and prevention has been desired.

A method for inhibiting the apoptosis-inducing activity of *Helicobacter pylori* is a very promising method. By neutralizing the apoptosis-inducing activity of the bacterium, apoptosis in gastric epithelial cells could be inhibited. Therefore, it is highly possible to suppress the development of gastritis and gastric ulcer.

To date, as the apoptosis-inducing substances produced by the bacterium, Cag A protein, urease, lipopolysaccharide (LPS), and vacuolus toxins have been studied (WO98/32467 etc.), however, the contribution of these substances in *Helicobacter pylori*-mediated apoptosis is rather small and the presence of other unknown factor has been expected for induction of the apoptosis (K. Shibayama et al., Infection and Immunity vol.69, No.5, 3181-3189 (2001)).

On the other hand, the total genome of *Helicobacter pylori* was determined in 1997 by genome project (Tomb J. F., et al., Nature Vol. 388,539-547,1997) and the whole nucleotide sequence has been registered in a database. However, the nucleotide sequence encoding the proteins, which have an apoptosis-inducing activity, has not been specified.

### Problems to be solved by the Invention

The objective of the present invention is to identify the apoptosis-inducing protein of the bacterium, to analyze the structure and to provide the amino acid sequence of the protein and the nucleotide sequence encoding the amino acid sequence. Further objective of the present invention is to provide inhibiting agents of an apoptosis-inducing activity of the bacterium for the treatment of the disease ascribed to the bacterium and a vaccine to prevent the infection of the bacterium.

### Means to solve the Problems

The inventors of the present invention succeeded in purifying and in identifying the protein with the apoptosis-inducing activity from *Helicobacter pylori* and accomplished the present invention. The apoptosis-inducing protein was found to be composed of two proteins. Analysis of the amino acid sequence of these two proteins and retrieval from the database indicated that both these proteins are encoded by the HP1118 gene. The gene is reported to encode γ - glutamyl transferase (Chevalier C., et al., Molecular Microbiology, vol. 31 1359-1372, 1999). However, the apoptosis-inducing activity of the protein has not examined in their report or in any other reports. Furthermore, γ-glutamyl transferase is a ubiquitous enzyme found throughout bacteria and various biological species, and functions to incorporate amino acids into cytoplasm. However, it has not been reported that the enzyme has the apoptosis-inducing activity.

In other words, the present invention demonstrated for the first time that a protein complex formed by association of two proteins, either of which is constituted by a part of the amino acid sequence encoded by γ-glutamyl transferase gene (HP1118), has an apoptosis-inducing activity of *Helicobacter pylori*.

Namely, the present invention is an apoptosis-inducing protein of *Helicobacter pylori* comprising two kinds of proteins having an amino acid sequence of SEQ ID NO: 1 or 2, or two kinds of proteins having an amino acid sequence of SEQ ID NO: 1 or 2 wherein one or several amino acids with respect to the amino acid sequence of SEQ ID NO: 1 or 2 is/are deleted, substituted or added, and having an apoptosis-inducing activity in human gastric adenocarcinoma cell line.

Furthermore, the present invention is a protein comprising either of the following proteins:
(a) a protein having an amino acid sequence of SEQ ID NO: 1.
(b) a protein having an amino acid sequence comprising a deletion, substitution or addition of one or several amino acids with respect to the amino acid sequence of SEQ ID NO: 1, and having an apoptosis-inducing activity in human gastric adenocarcinoma cell line by association with the protein comprising the amino acid sequence of SEQ ID NO: 2.

Moreover, the present invention is a protein comprising either of the following proteins:
(a) a protein having an amino acid sequence of SEQ ID NO: 2.
(b) a protein having an amino acid sequence comprising a deletion, substitution or addition of one or several amino acids with respect to the amino acid sequence of SEQ ID NO: 2, and having an apoptosis-inducing activity in human gastric adenocarcinoma cell line by association with the protein comprising the amino acid sequence of SEQ ID NO: 1.

In addition, the present invention is the apoptotic-inducing protein of *Helicobacter pylori* comprising said two proteins.

Still furthermore, the present invention is a vaccine comprising one of said proteins, wherein a part of the amino acid sequence of SEQ ID NO: 1 and 2 is deleted, substituted or added, and said protein has little or no apoptosis-inducing activity in gastric adenocarcinoma cell line and possesses a defensive effect against the infection of *Helicobacter pylori*.

The defensive effect of the infection could be examined by the following methods: The animal model for *Helicobacter pylori* infection has been established using Mongolian gerbil. Candidate protein for vaccination is administered to Mongolian gerbils by oral administration, transnasal administration, intramuscular injection or subcutaneous injection to induce immune response. Subsequently, live *Helicobacter pylori* are administered orally and the infection established is examined. If defensive effect against the infection is positive in the animal test, then the effect in human is examined in the same way.

Still moreover, the present invention are respective genes encoding said two kinds of proteins and a method for distinguishing between pathogenic strains and nonpathogenic strains of *Helicobacter pylori,* comprising a step of amplifying at least one of the whole or a part of said genes.

### Brief Description of the Drawings

Figure 1 shows the cytotoxic activity (the absorbance at 450 nm) of each fraction separated by a cation exchange column (HiTrap SP column, Pharmacia) in Example 1. The lower the value is, the fewer the viable cells are.
Figure 2 shows the cytotoxic activity (the absorbance at 450 nm) of each fraction separated by a hydrophobic column (HiTrap Phenyl column, Pharmacia) in Example 1. The lower the value is, the fewer the viable cells are.
Figure 3 shows the cytotoxic activity (the absorbance at 450 nm) of each fraction separated by a gel filtration column (Pharmacia) in Example 1. The lower the value is, the fewer the viable cells are.
Figure 4 shows the cytotocic activity (the absorbance at 450 nm) of each fraction separated by a cation exchange column (Mono S column, Pharmacia) in Example 1. The lower the value is, the fewer the viable cells are.
Figure 5 shows Native-PAGE profile of the purified apoptosis-inducing protein from *Helicobacter pylori*.
Figure 6 shows SDS-PAGE profile of the purified apoptosis-inducing protein from *Helicobacter pylori*.
Figure 7 shows the cytotoxic activity (the absorbance at 450 nm) of the purified apoptosis-inducing protein from *Helicobacter pylori* examined with AGS cells and HeLa cells. The lower the value is, the fewer the viable cells are.
Figure 8 shows the cytotoxic activity (the absorbance at 450 nm) of the purified apoptosis-inducing protein from *Helicobacter pylori* examined with KATO III cells. The lower the value is, the fewer the viable cells are.
Figure 9 shows the rate (%) of apoptotic cells of AGS cells induced by purified apoptosis-inducing protein from *Helicobacter pylori*.
Figure 10 shows the amino acid sequence of the protein (567 amino acid residues) transcribed from the HP1118 gene and the amino acid sequences of subunit 1 (SEQ ID NO: 1) and subunit 2 (SEQ ID NO: 2) of the apoptosis-inducing protein from *Helicobacter pylori.*
Figure 11 shows the schematic diagram of the apoptosis-inducing protein from *Helicobacter pylori.*

### Detailed Description of the Invention

Since the protein complex of the present invention, composed by thee association of two kinds of proteins, has the apoptosis-inducing activity of *Helicobacter pylori,* it can be used as the target molecule for the treatment of gastritis and stomach ulcer induced by *Helicobacter pylori.*

For example, the protein complex can be prepared either by introducing the genes encoding two subunits into expression vectors, e.g. in *E. coli*, separately and by mixing the subunits expressed independently, or by introducing a gene encoding both two subunits into an expression vector, e.g. in *E. coli* and by expressing these subunits at the same time.

After purification, the protein complex can be used to seek medical agents inhibiting an apoptosis-inducing activity. The apoptosis-inducing activity can be assayed by a conventional method for examining the apoptosis of gastric adenocarcinoma cell line.

The protein of the present invention can be applied to the development of a vaccine inhibiting the apoptosis-inducing activity of *Helicobacter pylori.*

For example, an animal could be immunized by an oral administration, transnasal administration or subcutaneous injection of the purified said protein complex as an antigen. Then, the animal is infected by the bacterium by an oral administration and is examined whether a defensive effect against the bacterium infection could be established. When the defensive effect in the animal is positive, the effect could be examined for humans. Furthermore, if there are common antigens between said protein complex and substances produced by human cells, the amino acid sequence of the common antigen could be examined and removed from said antigenic protein complex. A defensive effect against the infection could be examined by using the prepared protein, from which the common antigenic part is removed. Finally, the protein thus prepared, with a defensive effect, could be used as a vaccine.

Moreover, a mutation could be introduced into the gene encoding the apoptosis-inducing protein of *Helicobacter pylori* and the protein without the apoptosis-inducing activity, which is prepared from the modified gene, could be used as a vaccine.

Still furthermore, a novel *Helicobacter pylori* strain without the pathogenic activity could be prepared by disrupting the gene encoding the apoptosis-inducing protein of *Helicobacter pylori* and could be used as a vaccine.

Based on the nucleotide sequence of the gene encoding the apoptosis-inducing protein of *Helicobacter pylori* according to the present invention, it is possible to prepare PCR primers to amplify the gene. The PCR primers could be used to differentiate pathogenic *Helicobacter pylori* from nonpathogenic one.

The following examples illustrate this invention, however, it is not intended to limit the scope of this invention.

### Example 1

In this example, purification and identification of the apoptosis-inducing protein of *Helicobacter pylori* were conducted.

In this example, the apoptosis-inducing activity was measured as the cytotoxic activity in human gastric adenocarcinoma cell line AGS (ATCC CRL 1739) using Quick Cell Proliferation Assay Kit (Cat.BV-K301-10) (BioVision). The cell viability (surviving cell), from which an apoptosis-inducing activity is calculated, is based on the activity of intracellular mitochondrial dehydrogenase activity as a marker enzyme and is measured by the absorbance at 450 nm after 1 hr incubation at 37°C with the substrate of the enzyme. The absorbance indicates the cell viability. The more cytotoxic, the less the absorbance is according to the mesurement.

First of all, *Helicobacter pylori* strain 26695 (ATCC CTL 700392) was grown in 1000 ml of BHI medium (Becton Dikkinson Co., Brain Heart Infusion N0. 237500) supplemented with 10% serum. After harvest by centrifugation, the bacteria were suspended in PBS (Phosphate buffered saline, physiological saline (0.9% NaCl) adjusted to pH 7.4 with 50 mM sodium phosphate) and washed by centrifugation. The bacterial pellet was resuspended in 50 ml of PBS and disrupted by a French press at 120Mpa. After low speed centrifugation at 5000 x g for 10 min to remove the unbroken bacteria, membrane fraction was sedimented by centrifuging at 100,000 x g for 2 hrs. The membrane fractions were suspended in 50 mM phosphate buffer (pH 6.0) containing 1 M NaCl and again centrifuged at 100,000 x g for 2 hrs. During the procedures, the apoptosis-inducing protein was separated from membrane fractions and was solubilized in the supernatant fraction.

The membrane fractions were suspended in 50 mM phosphate buffer (pH 6.0) containing 1 M NaCl and again centrifuged at 100,000 x g for 2 hrs. During the procedures, the apoptosis-inducing protein was separated from membrane fractions and was solubilized in the supernatant fraction.

From the supernatant fraction, the apoptosis-inducing protein was purified in the following way.

In the beginning, the supernatant fraction was dialyzed against 250 mM NaCl-containing buffer, loaded in a cation exchange column (HiTrap SP column, Pharmacia), and fractionated by eluting with the gradient of 250-500 mM NaCl. Then, each fraction was assessed on the apoptosis-inducing activity.

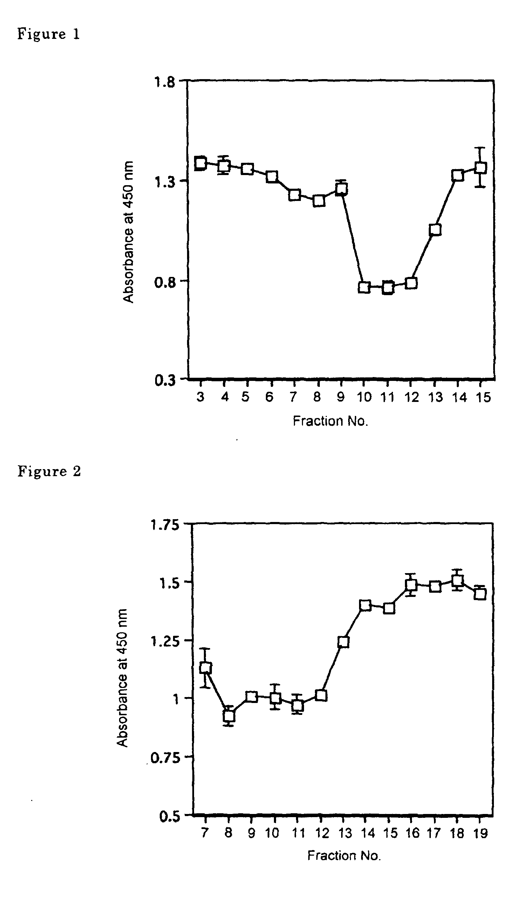

Figure 1 shows the result. Fractions 10 to 12 contained the activity. These fractions were collected and used for the subsequent purification steps. Then, the collected fraction containing the activity was loaded in a hydrophobic column (HiTrap Phenyl, Pharmacia) and fractionated by eluting with the gradient of 20-10% ammonium sulfate. Then, each fraction was assessed on the apoptosis-inducing activity. Figure 2 shows the results. Fractions 8 to 12 contained the activity. These fractions were collected and used for the following purification steps.

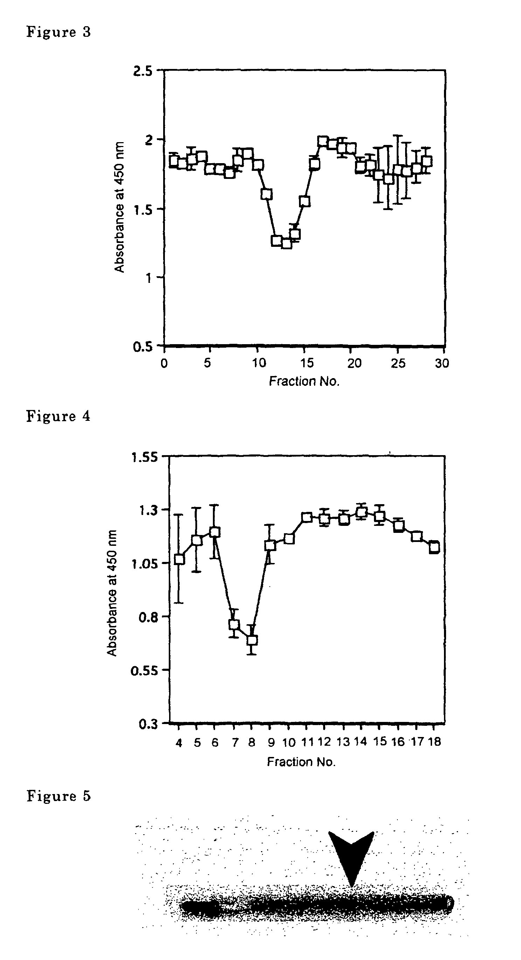

Then, the collected fraction containing the activity was loaded in a gel-filtration column (Pharmacia), fractionated by 50 mM phosphate buffer (pH 6.0) containing 500 mM NaCl and each fraction was assessed on the apoptosis-inducing activity. Figure 3 shows the results. Fractions 12 to 14 contained the activity. These fractions were collected and used for the following purification steps.

Then, the collected fraction containing the activity was dialyzed against 250 mM NaCl-containing buffer, loaded in a cation exchange column (MonoS column, Pharmacia) and fractionated by eluting with the gradient of 250-500 mM NaCl. Then, each fraction was assessed on the cytotoxic activity. Figure 4 shows the results. Fractions 7 and 8 contained the activity. These fractions were collected.

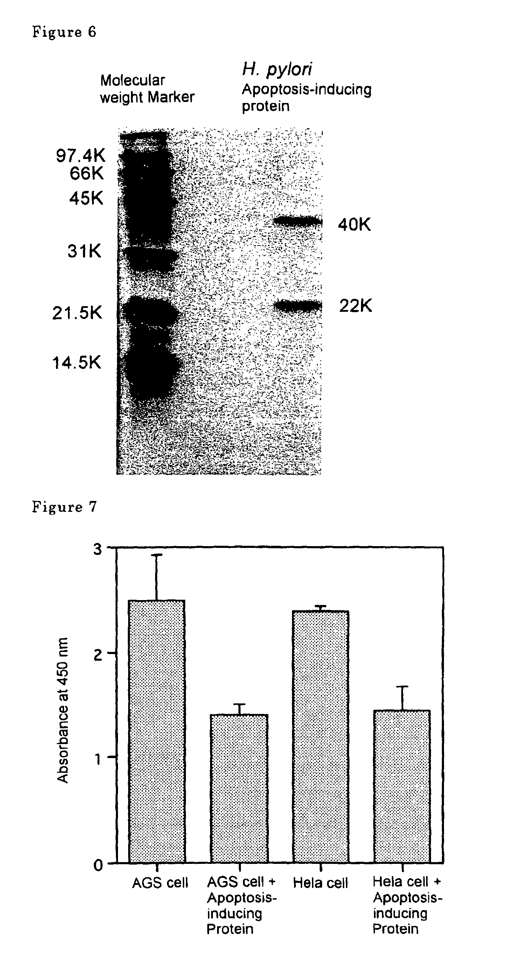

The purified protein obtained by the above procedures was analyzed by a native PAGE under acid condition. As shown in Fig. 5, a single band was observed. The single band was subsequently analyzed by 2D SDS-PAGE. As shown in Fig. 6, the band was separated into two bands (their molecular weight were about 40 kDa and 22 kDa).

In native gel, which is free from strong detergent such as SDS in acryl amide, the three dimensional structure of the protein is preserved. Observation of the single band indicates that the protein is composed of a single complex in its native state.

On the other hand, in SDS-PAGE, the three dimensional structure of the protein is disrupted by SDS and multiple protein subunits are separated. The result that a single band in native PAGE is separated into two bands in SDS-PAGE indicates that the protein complex is composed of the two protein subunits.

In other words, the apoptosis-inducing protein from *Helicobacter pylori* is a protein constructed by an association of two kinds of subunits.

### Example 2

In this example, the apoptosis-inducing activity of the protein purified in example 1 was examined in human gastric adenocarcinoma cell line AGS (ATCC CRL 1739), in human gastric carcinoma cell line KATO III (Human Science Research Resources Bank, Cat No. JCRB0611 (ATCC, HTB-103)) and in human uterine cervix cancer-derived HeLa cell (Human Science Research Resources Bank, Cat No. JCRB9004).

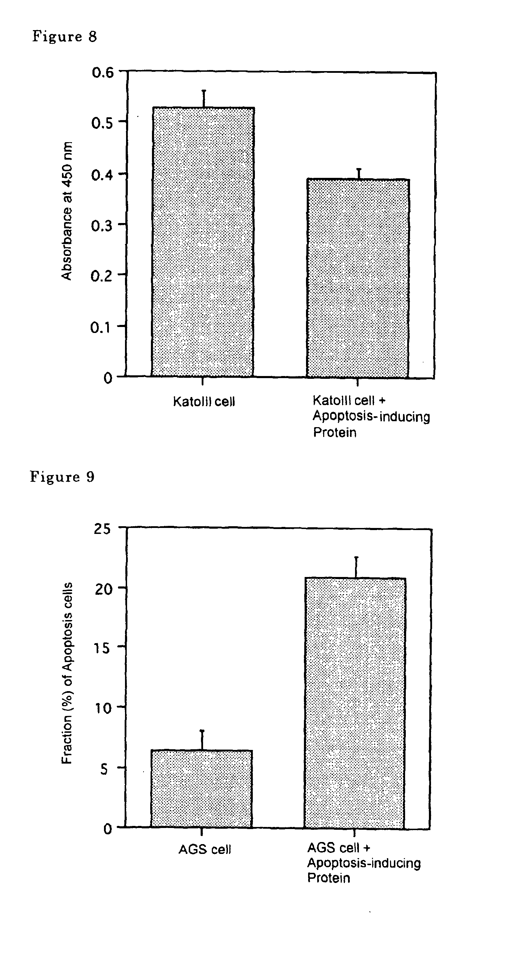

The cytotoxic effect was assayed using WST-1 kit (Roche Diagnotics). The results are shown in Figures 7 and 8.

As shown in these figures, the protein purified in example 1 has the cytotoxic activity in all the above cell lines.

Also, the induction of apoptosis in AGS cells by the protein was examined:
Trypsinized apoptotic and control cells were collected and stained with a fluorescent dye (Hoechst 33342), which stains cellular nuclei. Since the nuclei of apoptotic cells are highly condensed and fragmented, apoptotic cells are easily differentiated from control cells under fluorescence microscopy. Cells were put on a slide glass, the number of apoptotic cells and control cells were counted and the percentage of apoptotic cells in total cells was enumerated. At least total 500 cells were counted for each experiment.

The results are shown in Fig. 9. It was confirmed that the cytotoxic effect of the protein against AGS cells was mainly ascribed to apoptosis.

### Example 3

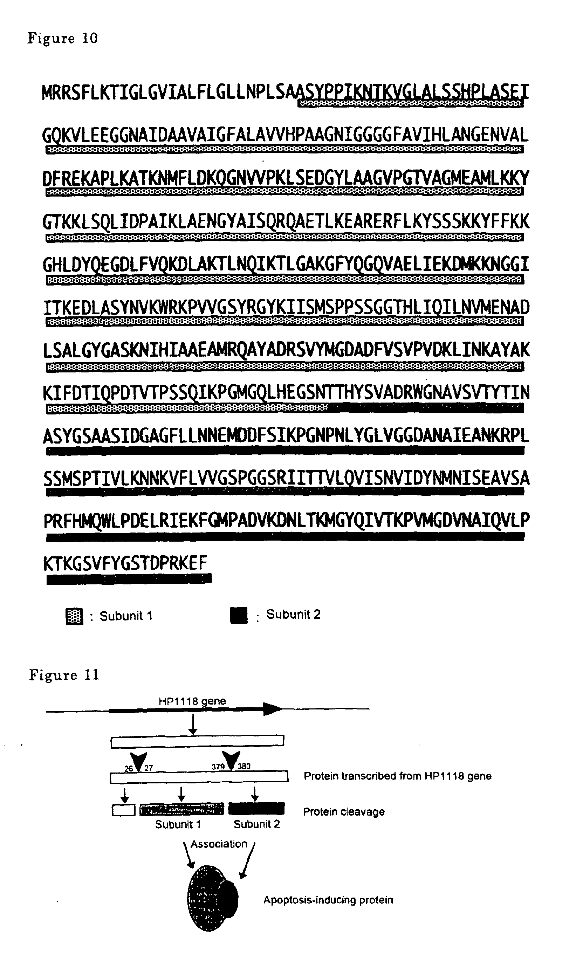

The amino acid sequencing of N-terminal end of the two subunits purified in example 1 indicated that both subunits were encoded by the HP1118 gene. Figure 10 shows the amino acid sequence of two subunits (subunits 1 and 2) together with that of the HP1118 gene. More specifically, the subunit 1 (SEQ ID NO: 1) and the subunit 2 (SEQ ID NO: 2) comprise amino acids 27-379 and 380-567, respectively, of the amino acid sequence encoded by the HP1118 gene.

As above described, the apoptosis-inducing protein of *Helicobacter pylori* is a protein complex composed of two protein subunits as shown in Fig. 11: one (subunit 1) is produced by the cleavage between 26^{th} and 27^{th} and between 379^{th} and 380^{th} of the amino acid residues of the protein transcribed from the HP1118 gene and the other (subunit 2) is produced by the cleavage between 379^{th} and 380^{th} of the amino acid residues of said protein.

## Claims

1. An apoptosis-inducing protein of *Helicobacter pylori* comprising two kinds of proteins having an amino acid sequence of SEQ ID NO: 1 or 2, or two kinds of proteins having an amino acid sequence of SEQ ID NO: 1 or 2 wherein one or several amino acids with respect to the amino acid sequence of SEQ ID NO: 1 or 2 is/are deleted, substituted or added, and having an apoptosis-inducing activity in human gastric adenocarcinoma cell line.

2. A protein comprising either of the following proteins:
(a) a protein having an amino acid sequence of SEQ ID NO: 1.
(b) a protein having an amino acid sequence comprising a deletion, substitution or addition of one or several amino acids with respect to the amino acid sequence of SEQ ID NO: 1, and having an apoptosis-inducing activity in human gastric adenocarcinoma cell line by association with the protein comprising the amino acid sequence of SEQ ID NO: 2.

3. A protein comprising either of the following proteins:
(a) a protein having an amino acid sequence of SEQ ID NO: 2.
(b) a protein having an amino acid sequence comprising a deletion, substitution or addition of one or several amino acids with respect to the amino acid sequence of SEQ ID NO: 2, and having an apoptosis-inducing activity in human gastric adenocarcinoma cell line by association with the protein comprising the amino acid sequence of SEQ ID NO: 1.

4. The apoptosis-inducing protein of *Helicobacter pylori* comprising the protein of claim 2 and the protein of claim 3.

5. A vaccine comprising the protein of claim 1 or 4, wherein a part of the amino acid sequence of SEQ ID NO: 1 and 2 is deleted, substituted or added, and said protein has little or no apoptosis-inducing activity in gastric adenocarcinoma cell line and possesses a defensive effect against the infection of *Helicobacter pylori.*

6. A gene encoding the protein of claim 2.

7. A gene encoding the protein of claim 3.

8. A method for distinguishing between pathogenic strains and nonpathogenic strains of *Helicobacter pylori*, comprising a step of amplifying at least one of the whole or a part of the genes of claims 6 and 7.


SEQUENCE LISTING

<110> Japan Science and Technology Agency

<120> An apoptosis-inducing protein from Helicobacter pylori

<130> FS03-319PCT

<160> 2

<210> 1

<211> 353

<212> PRT

<213> Helicobacter Pylori

<400> 1

Ala Ser Tyr Pro Pro Ile Lys Asn Thr Lys Val Gly Leu Ala Leu Ser

1               5                   10                  15

Ser His Pro Leu Ala Ser Glu Ile Gly Gln Lys Val Leu Glu Glu Gly

            20                  25                  30

Gly Asn Ala Ile Asp Ala Ala Val Ala Ile Gly Phe Ala Leu Ala Val

        35                  40                  45

Val His Pro Ala Ala Gly Asn Ile Gly Gly Gly Gly Phe Ala Val Ile

    50                  55                  60

His Leu Ala Asn Gly Glu Asn Val Ala Leu Asp Phe Arg Glu Lys Ala

65                  70                  75                  80

Pro Leu Lys Ala Thr Lys Asn Met Phe Leu Asp Lys Gln Gly Asn Val

                85                  90                  95

Val Pro Lys Leu Ser Glu Asp Gly Tyr Leu Ala Ala Gly Val Pro Gly

            100                 105                 110

Thr Val Ala Gly Met Glu Ala Met Leu Lys Lys Tyr Gly Thr Lys Lys

        115                 120                 125

Leu Ser Gln Leu Ile Asp Pro Ala Ile Lys Leu Ala Glu Asn Gly Tyr

    130                 135                 140

Ala Ile Ser Gln Arg Gln Ala Glu Thr Leu Lys Glu Ala Arg Glu Arg

145                 150                 155                 160

Phe Leu Lys Tyr Ser Ser Ser Lys Lys Tyr Phe Phe Lys Lys Gly His

                165                 170                 175

Leu Asp Tyr Gln Glu Gly Asp Leu Phe Val Gln Lys Asp Leu Ala Lys

            180                 185                 190

Thr Leu Asn Gln Ile Lys Thr Leu Gly Ala Lys Gly Phe Tyr Gln Gly

        195                 200                 205

Gln Val Ala Glu Leu Ile Glu Lys Asp Met Lys Lys Asn Gly Gly Ile

    210                 215                 220

Ile Thr Lys Glu Asp Leu Ala Ser Tyr Asn Val Lys Trp Arg Lys Pro

225                 230                 235                 240

Val Val Gly Ser Tyr Arg Gly Tyr Lys Ile Ile Ser Met Ser Pro Pro

                245                 250                 255

Ser Ser Gly Gly Thr His Leu Ile Gln Ile Leu Asn Val Met Glu Asn

            260                 265                 270

Ala Asp Leu Ser Ala Leu Gly Tyr Gly Ala Ser Lys Asn Ile His Ile

        275                 280                 285

Ala Ala Glu Ala Met Arg Gln Ala Tyr Ala Asp Arg Ser Val Tyr Met

    290                 295                 300

Gly Asp Ala Asp Phe Val Ser Val Pro Val Asp Lys Leu Ile Asn Lys

305                 310                 315                 320

Ala Tyr Ala Lys Lys Ile Phe Asp Thr Ile Gln Pro Asp Thr Val Thr

                325                 330                 335

Pro Ser Ser Gln Ile Lys Pro Gly Met Gly Gln Leu His Glu Gly Ser

            340                 345                 350

Asn

<210> 2

<211> 188

<212> PRT

<213> Helicobacter Pylori

<400> 2

Thr Thr His Tyr Ser Val Ala Asp Arg Trp Gly Asn Ala Val Ser Val

1               5                   10                  15

Thr Tyr Thr Ile Asn Ala Ser Tyr Gly Ser Ala Ala Ser Ile Asp Gly

            20                  25                  30

Ala Gly Phe Leu Leu Asn Asn Glu Met Asp Asp Phe Ser Ile Lys Pro

        35                  40                  45

Gly Asn Pro Asn Leu Tyr Gly Leu Val Gly Gly Asp Ala Asn Ala Ile

    50                  55                  60

Glu Ala Asn Lys Arg Pro Leu Ser Ser Met Ser Pro Thr Ile Val Leu

65                  70                  75                  80

Lys Asn Asn Lys Val Phe Leu Val Val Gly Ser Pro Gly Gly Ser Arg

                85                  90                  95

Ile Ile Thr Thr Val Leu Gln Val Ile Ser Asn Val Ile Asp Tyr Asn

            100                 105                 110

Met Asn Ile Ser Glu Ala Val Ser Ala Pro Arg Phe His Met Gln Trp

        115                 120                 125

Leu Pro Asp Glu Leu Arg Ile Glu Lys Phe Gly Met Pro Ala Asp Val

    130                 135                 140

Lys Asp Asn Leu Thr Lys Met Gly Tyr Gln Ile Val Thr Lys Pro Val

145                 150                 155                 160

Met Gly Asp Val Asn Ala Ile Gln Val Leu Pro Lys Thr Lys Gly Ser

                165                 170                 175

Val Phe Tyr Gly Ser Thr Asp Pro Arg Lys Glu Phe

            180                 185                 190